# FASCICULE DE BREVET EUROPEEN

(11) **EP 1 413 579 B1**
(45) Date de publication et mention de la délivrance du brevet: **02.02.2005**
(21) Numéro de dépôt: 03292634.7
(22) Date de dépôt: 22.10.2003
(51) Int. Cl.: C07D 409/06, C07D 403/06, C07D 401/06, A61K 31/417, A61K 31/381, A61K 31/402, A61K 31/44, A61P 3/00, A61P 9/10

(54) **Dérivés de l'imidazoline, leur procédé de préparation et les compositions pharmaceutiques qui les contiennent**
Imidazolinderivate, Verfahren zu ihrer Herstellung und diese enthaltende pharmazeutische Zusammensetzungen
Imidazoline derivatives, process for the preparation thereof and pharmaceutical compositions containing them

(30) Priorité: 23.10.2002 FR 0213194
(43) Date de publication de la demande: 28.04.2004
(73) Titulaire: LES LABORATOIRES SERVIER, 92415 Courbevoie Cédex (FR)
(72) Inventeur: Rault, Sylvain, 14370 Moult (FR); Kopp, Marina, 14000 Caen (FR); Lancelot, Jean-Charles, 14400 Le Bourg (FR); Lemaitre, Stéphane, 27720 Bois le Roi (FR); Caignard, Daniel-Henri, 78230 le Pecq (FR); Bizot-Espiard, Jean-Guy, 75015 Paris (FR); Renard, Pierre, 78150 Le Chesnay (FR)

(56) Documents cités:
- EP-A- 0 313 288
- EP-A- 0 413 433
- WO-A-93/09113
- GB-A- 1 259 005
- GB-A- 2 351 081
- BIHAN LE G ET AL: "Design and Synthesis of Imidazoline Derivatives Active on Glucose Homeostasis in a Rat Model of Type II Diabetes. 2. Syntheses an Biological Activities of 1,4-Dialkyl-, 1,4-Dibenzyl, and 1-Benzyl-4-alkyl-2-(4',5'-dihydro-1'H-imid azol-2'-yl)piperazines and Isosteric Analogs of Imidazoline" JOURNAL OF MEDICINAL CHEMISTRY, AMERICAN CHEMICAL SOCIETY. WASHINGTON, US, vol. 42, 1999, pages 1587-1603, XP002237953 ISSN: 0022-2623
- HAMLYN R J ET AL: "CARBON-CARBON BOND FORMATION VIA THERMAL INTERMOLECULAR HYDROGEN ATOM TRANSFER: TWO SERENDIPITOUS HETEROCYCLIC EXAMPLES" JOURNAL OF THE CHEMICAL SOCIETY, PERKIN TRANSACTIONS 1, CHEMICAL SOCIETY. LETCHWORTH, GB, vol. 12, 2000, pages 1811-1813, XP009009288 ISSN: 0300-922X

## Description

L'invention concerne de nouveaux dérivés de l'imidazoline, leur procédé de préparation ainsi que les compositions pharmaceutiques qui les contiennent.

Sur le plan des structures chimiques, la littérature fournit de très nombreux exemples de dérivés de l'imidazoline. Ils ont été notamment décrits pour leur utilisation en thérapeutique. Par exemples, des dérivés imidazoline sont connus comme ayant des propriétés cardiotoniques (GB 119/963), stimulantes α-adrénergiques (Eur. J. Med. Chem., 1989, 24(6), 619, J. Pharmacobio. Dyn., 1986, 9(4), 395), antidépressives et antiinflammatoires (US 3932-431), antihyperglycémiantes et antidiabétiques (EP 924209, EP 1145717, EP 288978, JP 04178381 et WO 0238559) ou pouvant traiter des pathologies liées aux récepteurs imidazoline (EP 846688).

La demanderesse a présentement découvert de nouveaux dérivés imidazoline de structures originale cycloalkylimidazoline lui conférant des propriétés antidiabétiques dépourvu d'effets secondaires, du fait de l'absence d'inhibition de recapture de sérotonine.

Plus particulièrement, la présente invention concerne les composés de formule (I) : 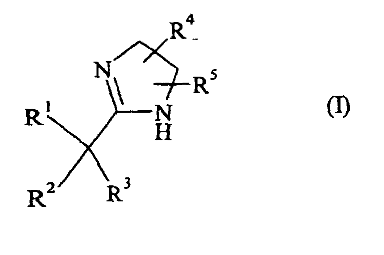 dans laquelle :
- R' représente un groupement hétéroaryle éventuellement substitué,
- R² représente un groupement cycloalkyle éventuellement substitué,
- R³ représente un atome d'hydrogène ou un groupement alkyle,
- R⁴ et R⁵, identiques ou différents, représentent un atome d'hydrogène, d'halogène ou un groupement alkyle, polyhalogénoalkyle, R¹⁰―C(X)―R¹¹―, R¹⁰―Y―C(X)―R¹¹―, R¹⁰―C(X)―Y―R¹¹―, R¹⁰―Y―R¹¹―ou R¹⁰―S(O)ₙ―R¹¹―,
   avec :
   - R¹⁰ représentant un atome d'hydrogène, ou un groupement alkyle,
   - R¹¹ représentant une liaison, ou un groupement alkylène, alkénylène ou alkynylène,
   - X représentant un atome d'oxygène, de soufre, ou un groupement NR¹² où R¹² représente un atome d'hydrogène ou un groupement alkyle,
   - Y représentant un atome d'oxygène, de soufre, ou un groupement amino ou alkylamino, et
   - n représentant un entier compris inclusivement entre 1 et 2,
leurs énantiomères, diastéréoisomères, tautomères, ainsi que leurs sels d'addition à un acide ou à une base pharmaceutiquement acceptable,
étant entendu que :
- le terme "alkyle" désigne une chaîne hydrocarbonée, linéaire ou ramifiée, contenant de 1 à 6 atomes de carbone,
- le terme "alkoxy" désigne un groupement alkyle-oxy dont la chaîne alkyle, linéaire ou ramifiée, contient de 1 à 6 atomes de carbone,
- le terme "alkylène" désigne une chaîne hydrocarbonée, linéaire ou ramifié, bivalente, contenant de 1 à 6 atomes de carbone,
- le terme "alkénylène" désigne une chaîne hydrocarbonée, linéaire ou ramifiée, bivalente, contenant de 1 à 6 atomes de carbone et de 1 à 3 doubles liaisons,
- le terme "alkynylène" désigne une chaîne hydrocarbonée, linéaire ou ramifiée, bivalente, contenant de 1 à 6 atomes de carbone et de 1 à 3 triples liaisons,
- le terme "polyhalogénoalkyle" désigne une chaîne carbonée, linéaire ou ramifiée, contenant de 1 à 3 atomes de carbone et de 1 à 7 atomes d'halogène,
- le terme "hétéroaryle" désigne un groupement de 5 à 11 chaînons, monocyclique ou bicyclique, dans lequel au moins un des cycles est aromatique, comportant dans le monocycle ou dans le bicycle 1, 2 ou 3 hétéroatomes, choisis parmi l'azote, l'oxygène et le soufre, et
- le terme cycloalkyle désigne un monocycle ou bicycle hydrocarboné comportant de 3 à 10 atomes de carbone, et éventuellement insaturé par 1ou 2 insaturations,
- le terme "éventuellement substitué" associé aux expressions cycloalkyle et hétéroaryle signifie que les groupements concernés sont non substitués ou substitués par un ou deux substituants, identiques ou différents, choisis parmi les atomes d'halogène et les groupements alkyle, alkoxy, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que le groupement hétéroaryle peut être en plus substitué par un groupement oxo sur la partie non-aromatique de l'hétéroaryle.

Parmi les acides pharmaceutiquement acceptables, on peut citer à titre non limitatif, les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif, l'hydroxyde de sodium, l'hydroxyde de potassium, la triéthylamine, etc...

Un aspect avantageux de l'invention concerne les composés pour lesquels R⁴ et R⁵, identiques ou différents, représentent un atome d'hydrogène ou un groupement alkyle.

Un autre aspect avantageux concerne les composés de formule (I) pour lesquels R³ représente un atome d'hydrogène.

Le groupement alkyle préféré de l'invention pour les groupements R⁴ et/ou R⁵ est le groupement méthyle.

Les groupements R² préférés de l'invention sont les groupements cyclopentyle, cyclohexyle et cycloheptyle, éventuellement substitué par un groupement alkyle, et plus particulièrement le groupement cyclohexyle.

Les groupements hétéroaryle préférés de l'invention sont les groupements aromatiques à 5 ou 6 chaînons tels que les groupements furanyle, thiènyle, pyrrolyle, ou pyridyle et plus particulièrement thiènyle.

Un aspect particulièrement avantageux de l'invention concerne les composés de formule (I) pour lesquels, R' représente un groupement hétéroaryle à 5 ou 6 chaînons éventuellement substitués, R² représente un groupement cyclohexyle ou cycloheptyle éventuellement substitué par un groupement alkyle, R³ représente un atome d'hydrogène, R⁴ et R⁵, identiques ou différents, représentent un atome d'hydrogène ou un groupement alkyle.

Parmi les composés préférés de l'invention, on peut citer plus particulièrement le 2-[cyclohexyl(3-thiènyl)méthyl]-4-méthyl-4,5-dihydro-1*H*-imidazole et son tautomère le 2-[cyclohexyl(3-thiènyl)méthyl]-5-méthyl-4,5-dihydro-1*H*-imidazole, le (4*S*)-2-[cyclohexyl(3-thiènyl)méthyl]-4-méthyl-4,5-dihydro-1*H*-imidazole et son tautomère le (4*S*)-2-[cyclohexyl(3-thiènyl)méthyl]-5-méthyl-4,5-dihydro-1*H*-imidazole, le (4*R*)-2-[cyclohexyl(3-thiènyl)méthyl]-4-méthyl-4,5-dihydro-1*H*-imidazole et son tautomère le (4*R*)-2-[cyclohexyl(3-thiènyl)méthyl]-5-méthyl-4,5-dihydro-1*H*-imidazole.

L'invention concerne également le procédé de préparation des composés de formule (I), caractérisé en ce que l'on utilise comme produit de départ un composé de formule (II) : 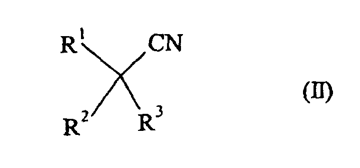 dans laquelle R¹, R² et R³ sont tels que définis dans la formule (I),
composés de formule (II) qui se condensent à la diamine (III) : 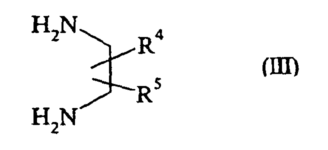 dans laquelle R⁴ et R⁵ sont tels que définis dans la formule (I)
pour conduire en présence de catalyseur approprié aux composés de formule (I),
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu
- qu'à tout moment jugé opportun au cours du procédé précédemment décrit, le ou les groupements carbonyles, thiocarbonyles, amino, alkylamino des réactifs de départ (II) et (III) peuvent être protégés puis, après condensation, déprotégés pour les besoins de la synthèse,
- que les réactifs (II), et (III) sont préparés selon des modes opératoires connus, décrits dans la littérature.

Les composés montrent notamment une excellente activité dans la réduction des taux de glucose sanguin. Ces propriétés justifient leur application en thérapeutique dans le traitement et/ou la prophylaxie des hyperglycémies, dyslipidémies, et plus particulièrement dans le traitement des diabètes non insulino dépendants de type II, de l'obésité, de l'intolérance au glucose, et des complications diabétiques en particulier au niveau cardiovasculaire.
L'activité de ces composés est également recommandée pour le traitement et/ou la prophylaxie d'autres maladies incluant le diabète de type I, les hypertriglycéridémies, le syndrome métabolique, la résistance à l'insuline, les dyslipidémies chez le diabétique, les hyperlipidémies et l'hypercholestérolémie.

Les composés de la présente invention possédant de surcroît une très faible affinité sur les sites de récepteur de la sérotonine, ils sont faiblement toxiques, contrairement à ceux de l'art antérieur.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon. Les structures des composés décrits ont été confirmées par des techniques spectroscopiques et spectrométriques usuelles.

Les produits de départ utilisés sont des produits connus ou préparés selon des modes opératoires connus.

Le nom des composés exemplifiés ci après sous-entend aussi leur tautomère, par exemple le 2-[cyclohexyl(3-thiènyl)méthyl]-4-méthyl-4,5-dihydro-1*H*-imidazole et son tautomère le 2-[cyclohexyl(3-thiènyl)méthyl]-5-méthyl-4,5-dihydro-1*H*-imidazole.

### PROTOCOLE GENERAL A : synthèse des dérivés cycloalkylidène acétonitriles (PREPARATIONS 1 à 6) :

On ajoute 0,1 mole de dérivé acétonitrile à une solution de 0,1 mole d'éthylate de sodium dans 50 ml d'éthanol puis on introduit 0,1 mole de dérivé cétonique. Le mélange est chauffé à 60°C pendant 4 heures, la solution obtenue est ensuite concentrée sous pression réduite jusqu'à la moitié du volume initial puis versée dans 200 ml d'eau. Après extraction à l'éther, les fractions éthérées sont lavées à l'eau, séchées sur sulfate de magnésium et le solvant est évaporé. Le produit ainsi obtenu sera utilisé dans l'étape suivante sans purification supplémentaire.

### PROTOCOLE GENERAL B : réduction des composés nitrés (PREPARATIONS 7 à 12)

On ajoute à une solution de 0,05 mole de composés de Préparation 1-6 dans 100 ml d'éthanol, 0,125 mole de borohydrure de sodium. La solution est chauffée au reflux de l'éthanol pendant 24 heures, l'éthanol est évaporé sous pression réduite. Le résidu est repris dans 500 ml d'eau et extrait à l'éther. Le produit souhaité est obtenu après séchage sur sulfate de magnésium de la phase organique et évaporation de l'éther. Le produit ainsi obtenu sera utilisé dans l'étape suivante sans purification supplémentaire.

### PREPARATION 1 : Cyclopentylidène(3-thiènyl)acétonitrile

On obtient l'intermédiaire attendu à partir du 3-thiènylacétonitrile et de la cyclopentanone, comme décrit dans le protocole général A.

### PREPARATION 2 : Cyclohexylidène(2-thiènyl)acétonitrile

On obtient l'intermédiaire attendu à partir du 2-thiènylacétonitrile et de la cyclohexanone, comme décrit dans le protocole général A.

### PREPARATION 3 : Cyclohexylidène(3-thiènyl)acétonitrile

On obtient l'intermédiaire attendu à partir du 3-thiènylacétonitrile et de la cyclohexanone, comme décrit dans le protocole général A.

### PREPARATION 4 : (4-Méthylcyclohexylidène)(3-thiènyl)acétonitrile

On obtient l'intermédiaire attendu à partir du 3-thiènylacétonitrile et de la 4-méthylcyclohexanone, comme décrit dans le protocole général A.

### PREPARATION 5 : Cycloheptylidène(3-thiènyl)acétonitrile

On obtient l'intermédiaire attendu à partir du 3-thiènylacétonitrile et de la cycloheptanone, comme décrit dans le protocole général A.

### PREPARATION 6 : Cyclobexylidène(1-méthyl-1H-pyrrol-2-yl)acétonitrile

On obtient l'intermédiaire attendu à partir du (1-méthyl-1*H*-pyrrol-3-yl)acétonitrile et de la cyclohexanone, comme décrit dans le protocole général A.

### PREPARATION 7 : Cyclopentyl(3-thiènyl)acétonitrile

On obtient l'intermédiaire attendu à partir du composé de la préparation 1, comme décrit dans le protocole général B.

### PREPARATION 8 : Cyclohexyl(2-thiènyl)acétonitrile

On obtient l'intermédiaire attendu à partir du composé de la préparation 2, comme décrit dans le protocole général B.

### PREPARATION 9 : Cyclohexyl(3-thiènyl)acétonitrite

On obtient l'intermédiaire attendu à partir du composé de la préparation 3, comme décrit dans le protocole général B.

### PREPARATION 10 : (4-Méthylcyclohexyl)(3-thiènyl)acétonitrile

On obtient l'intermédiaire attendu à partir du composé de la préparation 4, comme décrit dans le protocole général B.

### PREPARATION 11 : Cycloheptyl(3-thiènyl)acétonitrile

On obtient l'intermédiaire attendu à partir du composé de la préparation 5, comme décrit dans le protocole général B.

### PREPARATION 12 : Cyclohexyl(1-méthyl-1H pyrrol-2-yl)acétonitrile

On obtient l'intermédiaire attendu à partir du composé de la préparation 6, comme décrit dans le protocole général B.

### PREPARATION 13 : Cyclohexyl(pyridin-2-yl)acétonitrile

On ajoute 0,1 mole de 2-pyridylacétonitrile dans 10 ml de benzène à une suspension de 0,011 mole d'amidure de sodium dans 15 ml de benzène anhydre. Le milieu réactionnel est chauffé à reflux pendant 3 heures. On laisse ensuite revenir la solution à température ambiante pour pouvoir additionner goutte à goutte 0,1 mole de bromocyclohexane puis la solution est de nouveau portée à reflux pendant 8 heures.
Le mélange est refroidi et 50 ml d'eau sont additionnés pour éliminer l'excès d'amidure de sodium. La phase organique est extraite, séchée sur sulfate de magnésium et le solvant est évaporé sous pression réduite.

### PROTOCOLE GENERAL C : synthèse des composés des EXEMPLES 1 à 12 :

On dissout 0,02 mole composés des préparations 7 à 13 dans 25 ml du dérivé diaminé choisi, on ajoute une quantité catalytique de pentasulfure de phosphore (0,5 g, 0,001 mole). Le mélange est porté à reflux pendant 5 heures. La solution des ensuite versée dans 50 ml d'eau et extraite avec 2 fois 50 ml de dichlorométhane. Après évaporation du solvant, le produit est recristallisé dans un minimum d'acétonitrile.

### EXEMPLE 1 : 2-[Cyclopentyl(3-thiènyl)méthyl]-4,5-dihydro-1H-imidazole

On obtient le composé du titre à partir du composé de la Préparation 7, et de la 1,2-éthanediamine, suivant le protocole général C.
*Point de fusion : 157°C*.

### EXEMPLE 2 : 2-[Cyclohexyl(2-thiènyl)méthyl]-4,5-dihydro-1H-imidazole

On obtient le composé du titre à partir du composé de la Préparation 8, et de la 1,2-éthanediamine, suivant le protocole général C.
*Point de fusion : 155°C*.

### EXEMPLE 3 : 2-[Cyclohexyl(3-thiényl)méthyl]-4,5-dihydro-1H-imidazole

On obtient le composé du titre à partir du composé de la Préparation 9, et de la 1,2-éthanediamine, suivant le protocole général C.
*Point de fusion : 182°C*.

### EXEMPLE 4 : 2-[Cyclobexyl(3-thiènyl)méthyl]-4,5-dihydro-1H-imidazole, énantiomère 1

En séparant par chromatographie chirale le composé précédemment décrit dans l'exemple 3, un des énantiomères est isolé.

### EXEMPLE 5 : 2-[Cyclohexy](3-thiènyl)méthyl]-4,5-dibydro-1H-imidazole, énantiomère 2

En séparant par chromatographie chirale le composé précédemment décrit dans l'exemple 3, l'autre énantiomère est isolé.

### EXEMPLE 6 : 2-[Cyclobexyl(3-thiènyl)méthyl]-4-méthyl-4,5-dihydro-1H-imidazole

On obtient le composé du titre à partir du composé de la Préparation 9, et de la 1,2-propanediamine, suivant le protocole général C.
*Point de fusion : 155°C.*

### EXEMPLE 7 : 2-(Cyclohexyl(3-thiènyl)méthyl]-4,4-diméthyl-4,5-dihydro-1H-imidazole

On obtient le composé du titre à partir du composé de la Préparation 9, et de la 2-méthyl-1,2-propanediamine, suivant le protocole général C.
*Point de fusion : 162°C.*

### EXEMPLE 8 : 2-[(4-Méthylcyclohexyl)(3-thiènyl)méthyl]-4,5-dihydro-1H-imidazole

On obtient le composé du titre à partir du composé de la Préparation 10, et de la 1,2-éthanediamine, suivant le protocole général C.
*Point de fusion : 168°C*.

### EXEMPLE 9 : 2-[Cyaohexyl(1-méthyl-1H-pyrrol-2-yl)méthyl]-4,5-dihydro-1H-imidazole

On obtient le composé du titre à partir du composé de la Préparation 12, et de la 1,2-éthanediamine, suivant le protocole général C.
*Point de fusion : 120°C*.

### EXEMPLE 10 :2-[Cyclohexyl(4,5-dihydro-1H imidazol-2-yl)méthyl]pyridine

On obtient le composé du titre à partir du composé de la Préparation 13, et de la 1,2-éthanediamine, suivant le protocole général C.
Point de fusion : *116°C*.

### EXEMPLE 11 : 2-[Cycloheptyl(3-thiènyl)méthyl]-4,5-dihydro-1H-imidazole

On obtient le composé du titre à partir du composé de la Préparation 11, et de la 1,2-éthanediamine, suivant le protocole général C.
*Point de fusion : 155°C*.

### EXEMPLE 12 : 2-[Cycloheptyl(3-thiènyl)méthyl]-4-méthyl-4,5-dihydro-1H-imidazole

On obtient le composé du titre à partir du composé de la Préparation 11, et de la 1,2-propanediamine, suivant le protocole général C.
*Point de fusion : 125°C.*

### EXEMPLE 13 : (4S)-2-(Cyclobexyl(3-thiènyl)méthyl]-4-méthyl-4,5-dihydro-1H-imidazole

On obtient le composé du titre à partir du composé de la Préparation 9, et de la (2*S*) 1,2-propanediamine, suivant le protocole général C.
*Point de fusion : 153°C*.

### EXEMPLE 14 : (4R)-2-[Cyclohexyl(3-thiènyl)méthyl]-4-méthyl-4,5-dihydro-1H-imidazole

On obtient le composé du titre à partir du composé de la Préparation 9, et de la (2*R*) 1,2-propanediamine, suivant le protocole général C.
*Point de fusion : 154°C*.

### ETUDE PHARMACOLOGIOUE

### EXEMPLE A : Activité hypoglycémiante

L'activité hypoglycémiante des dérivés de l'invention a été recherchée sur des rats mâles Witsar d'environ 250 g âgés de trois mois. Un diabète expérimental est obtenu par injection *iv* d'une faible dose de streptozotocine (35 mg/kg *iv*) dissoute dans un tampon citrate sous anesthésie au chlorhydrate de Kétamine. Ces rats sont appelés "STZ", ils sont caractérisés par une légère hyperglycémie basale, une nette intolérance au glucose et une franche altération de la sécrétion d'insuline.

L'homéostasie a été évaluée par un test de tolérance au glucose, réalisée deux semaines après injection de streptozotocine.
Enfin l'activité hypoglycémiant a été évalué sur des rats "Zucker". Les rats "Zucker fatty" fa/fa sont apparus à la suite d'une mutation spontanée dans la souche 13 M (Zucker & Zucker, 1961), et sont génétiquement insulinorésistants et obèses.
Leur obésité est observable dès l'âge de quatre semaines, elle s'accompagne donc d'insulinorésistance, d'une hyperinsulinémie, et d'une hyperlipidémie. Ce modèle est prédictifs des états diabétiques présentant des désordres métaboliques associés tel que l'obésité.
L'homéostasie a été évaluée également par un test de tolérance au glucose.

### . Test de tolérance au glucose par voie orale (OGTT)

Le glucose est administré *per os* (2g/kg) à des rats vigiles. Les échantillons de sang sont collectés avant et 10, 20, 30, 40, 60, 90 et 120 minutes après l'administration du glucose.

Le produit à tester est administré *per os* 1 heure avant l'OGTT, et les animaux témoins reçoivent le solvant (gomme arabique).

Les composés de l'invention diminuent très significativement la glycémie.

Par exemple, à 10 mg/kg le composé de l'Exemple 6 diminue la glycémie de 13 %, 18 % et 14 % sur les rats Wistar non diabétique, sur les rats Wistar STZ et sur les rats Zucker respectivement.

### EXEMPLE B : Activité hypolipémiante

Les produits de l'invention ont été testés *in vivo* chez la souris obèse ob/ob, utilisé comme modèle d'insulinorésistance associée à l'obésité. A titre d'exemple, le composé de l'Exemple 6 baisse significativement les triglycérides (après administration chronique par voie orale à la dose de 30 mg/kg/jour pendant 4 jours) de 32 % (animaux traités avec le composé de l'Exemple 6 vs. animaux non traités).

Dans ce modèle, les composés de l'invention se sont donc révélés être aussi de puissants hypolipémiants.

### EXEMPLE C : Détermination de l'affinité pour les sites de recapture de la sérotonine chez le rat

L'affinité des composés de l'invention a été déterminée par des expériences de compétition avec le [³H]-paroxetine. Les membranes sont préparées à partir de cortex frontal de rat et incubées en triple avec 0.25 nM de [³H]-paroxetine et le ligand froid dans un volume final de 0.4 ml, pendant 2 heures à 25°C. Le tampon d'incubation contient 50 mM de TRIS-HCl (pH 7,4), 120 mM de NaCl et 5 mM de KCl. La fixation non-spécifique est déterminée avec 10 µM de citalopram. A la fin de l'incubation, le milieu est filtré au travers de filtres et lavés trois fois avec 5 ml de tampon refroidit. La radioactivité retenue sur les filtres est déterminée par comptage du liquide de scintillation. Les isothermes de binding sont analysées par régression non-linéaire pour déterminer les valeurs d'IC₅₀.

Il apparaît que les composés de l'invention possèdent une plus faible affinité pour les sites de recapture de la sérotonine que ceux de l'art antérieur, et donc une diminution de la toxicité centrale confirmée par le test d'Irwin (voir Exemple D).

A titre d'exemple, le composé de l'Exemple 3 possède une IC₅₀ de 2.10⁻⁶ M, supérieure à celle de l'art antérieur (EP 846688) : du 2-[cyclohexyl(phényl)méthyl]-4,5-dihydro-1H-imidazole (1,5.10⁻⁷ M).

### EXEMPLE D : Etude de toxicité aigüe - Test d'Irwin

Trois rats par dose sont traités *per os* avec un des composés de l'invention (dispersé dans 0,5 % de carboxyméthylcellulose dans l'eau distillée) et sont observés à des intervalles de temps réguliers après 24 heures. La présence ou l'absence des symptômes sont enregistrés : mortalité, sédation, excitation, agressivité, forme de la queue, convulsions, douleurs, tremblements, exophtalmie, salivation, piloérection, défécation, peur, etc, suivant les critères décrits par Irwin (Psychopharmacologia, 1968, 13, 222). Ce test permet une évaluation de la toxicité et de l'effet sur le comportement.

Il apparaît que les composés de l'invention, d'après l'index thérapeutique (rapport de la dose minimale active sur la dose d'apparition des signes au niveau du système nerveux central), sont moins toxiques que ceux ce l'art antérieur.

### EXEMPLE E : Composition pharmaceutique

| Formule de préparation pour 1000 comprimés dosés à 10 mg: | |
|---|---|
| Composé de l'exemple 6 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 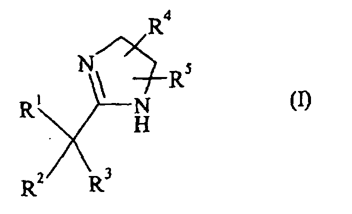 dans laquelle :
• R¹ représente un groupement hétéroaryle éventuellement substitué,
• R² représente un groupement cycloalkyle éventuellement substitué,
• R³ représente un atome d'hydrogène ou un groupement alkyle,
• R⁴ et R⁵, identiques ou différents, représentent un atome d'hydrogène, d'halogène ou un groupement alkyle, polyhalogénoalkyle, R¹⁰―C(X)―R¹¹―, R¹⁰―Y―C(X)―R¹¹― , R¹⁰―C(X)―Y―R¹¹―, R¹⁰―Y―R¹¹―ou R¹⁰―S(O)ₙ―R¹¹―,
avec :
- R¹⁰ représentant un atome d'hydrogène, ou un groupement alkyle,
- R¹¹ représentant une liaison, ou un groupement alkylène, alkénylène ou alkynylène,
- X représentant un atome d'oxygène, de soufre, ou un groupement NR¹² où R¹² représente un atome d'hydrogène ou un groupement alkyle,
- Y représentant un atome d'oxygène, de soufre, ou un groupement amino ou alkylamino, et
- n représentant un entier compris inclusivement entre 1 et 2,
leurs énantiomères, diastéréoisomères, tautomères, ainsi que leurs sels d'addition à un acide ou à une base pharmaceutiquement acceptable,
étant entendu que :
- le terme "alkyle" désigne une chaîne hydrocarbonée, linéaire ou ramifiée, contenant de 1 à 6 atomes de carbone,
- le terme "alkoxy" désigne un groupement alkyle-oxy dont la chaîne alkyle, linéaire ou ramifiée, contient de 1 à 6 atomes de carbone,
- le terme "alkylène" désigne une chaîne hydrocarbonée, linéaire ou ramifié, bivalente, contenant de 1 à 6 atomes de carbone,
- le terme "alkénylène" désigne une chaîne hydrocarbonée, linéaire ou ramifiée, bivalente, contenant de 1 à 6 atomes de carbone et de 1 à 3 doubles liaisons,
- le terme "alkynylène" désigne une chaîne hydrocarbonée, linéaire ou ramifiée, bivalente, contenant de 1 à 6 atomes de carbone et de 1 à 3 triples liaisons,
- le terme "polyhalogénoalkyle" désigne une chaîne carbonée, linéaire ou ramifiée, contenant de 1 à 3 atomes de carbone et de 1 à 7 atomes d'halogène,
- le terme "hétéroaryle" désigne un groupement de 5 à 11 chaînons, monocyclique ou bicyclique, dans lequel au moins un des cycles est aromatique, comportant dans le monocycle ou dans le bicycle 1, 2 ou 3 hétéroatomes, choisis parmi l'azote, l'oxygène et le soufre, et
- le terme cycloalkyle désigne un monocycle ou bicycle hydrocarboné comportant de 3 à 10 atomes de carbone, et éventuellement insaturé par 1ou 2 insaturations,
- le terme "éventuellement substitué" associé aux expressions cycloalkyle et hétéroaryle signifie que les groupements concernés sont non substitués ou substitués par un ou deux substituants, identiques ou différents, choisis parmi les atomes d'halogène et les groupements alkyle, alkoxy, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que le groupement hétéroaryle peut être en plus substitué par un groupement oxo sur la partie non-aromatique de l'hétéroaryle.

2. Composé de formule (I) selon la revendication 1 pour lesquels R⁴ et R⁵, identiques ou différents, représentent un atome d'hydrogène ou un groupement alkyle, leurs énantiomères, diastéréoisomères, tautomères, ainsi que leurs sels d'addition à un acide ou une base pharmaceutiquement acceptable. 18

3. Composé de formule (I) selon une quelconque des revendications 1 ou 2 pour lesquels R³ représente un atome d'hydrogène, leurs énantiomères, diastéréoisomères, tautomères, ainsi que leurs sels d'addition à un acide ou une base pharmaceutiquement acceptable.

4. Composé de formule (I) selon une quelconque des revendications 1 à 3 pour lesquels R¹ représente un groupement hétéroaryle à 5 ou 6 chaînons, éventuellement substitué, leurs énantiomères, diastéréoisomères, tautomères, ainsi que leurs sels d'addition à un acide ou une base pharmaceutiquement acceptable.

5. Composé de formule (I) selon une quelconque des revendications 1 à 4 pour lesquels R² représente un groupement cyclopentyle, cyclohexyle ou cycloheptyle, éventuellement substitué par un groupement alkyle, leurs énantiomères, diastéréoisomères, tautomères, ainsi que leurs sels d'addition à un acide ou une base pharmaceutiquement acceptable.

6. Composé de formule (I) selon une quelconque des revendications 1 à 5 pour lesquels R¹ représente un groupement hétéroaryle à 5, ou 6 chaînons, éventuellement substitué, R² représente un groupement cyclohexyle ou cycloheptyle éventuellement substitué par un groupement alkyle, R³ représente un atome d'hydrogène, et R⁴ et R⁵, identiques ou différents, représentent un atome d'hydrogène ou un groupement alkyle, leurs énantiomères, diastéréoisomères, tautomères, ainsi que leurs sels d'addition à un acide ou une base pharmaceutiquement acceptable.

7. Composé de formule (I) selon une quelconque des revendications 1, 2, 5 ou 6 pour lesquels le groupement alkyle représente un groupement méthyle, leurs énantiomères, diastéréoisomères, tautomères, ainsi que leurs sels d'addition à un acide ou une base pharmaceutiquement acceptable.

8. Composé de formule (I) selon une quelconque des revendications 1 à 7 qui est le 2-[cyclohexy](3-thiènyl)méthyl]-4-méthyl-4,5-dihydro-1*H*-imidazole, ses énantiomères, diastéréoisomères, tautomères ainsi que les sels d'addition à un acide pharmaceutiquement acceptable. 19

9. Composé de formule (I) selon une quelconque des revendications 1 à 7 qui est le (4*S*)-2-[cyclohexyl(3-thiènyl)méthyl]-4-méthyl-4,5-dihydro-1*H*-imidazole, ses diastéréoisomères, tautomères ainsi que les sels d'addition à un acide pharmaceutiquement acceptable.

10. Composé de formule (I) selon une quelconque des revendications 1 à 7 qui est le (4*R*)-2-[ cyclohexyl(3-thiènyl)méthyl]-4-méthyl-4,5-dihydro-1*H*-imidazole, ses diastéréoisomères, tautomères ainsi que les sels d'addition à un acide pharmaceutiquement acceptable.

11. Procédé de préparation des composés de formule (I) selon la revendication 1, **caractérisé en ce que** l'on utilise comme produit de départ un composé de formule (II) : 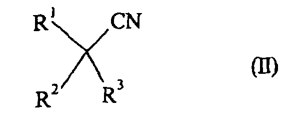 dans laquelle R¹, R² et R³ sont tels que définis dans la formule (I),
composés de formule (II) qui se condensent à la diamine (III) : 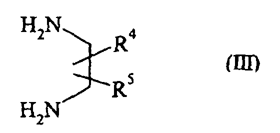 dans laquelle R⁴ et R⁵ sont tels que définis dans la formule (I)
pour conduire en présence de catalyseur approprié aux composés de formule (I),
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu
- qu'à tout moment jugé opportun au cours du procédé précédemment décrit, le ou les groupements carbonyles, thiocarbonyles, amino, alkylamino des réactifs de départ (II) et (III) peuvent être protégés puis, après condensation, déprotégés pour les besoins de la synthèse,
- que les réactifs (II), et (III) sont préparés selon des modes opératoires connus, décrits dans la littérature.

12. Composition pharmaceutique contenant comme principe actif au moins un composé selon une quelconque des revendications 1 à 10, seul ou en combinaison avec un ou plusieurs excipients ou véhicules inertes, non toxiques, pharmaceutiquement acceptables.

13. Composition pharmaceutique selon la revendication 12 contenant au moins un principe actif selon l'une quelconque des revendications 1 à 10 utiles pour la fabrication de médicaments traitant le diabète de type II non insulinodépendant, l'obésité, le diabète de type I, l'hyperlipidémie, l'hypercholestérolémie et leurs complications cardiovasculaires.

14. Composition pharmaceutique selon la revendication 12 contenant au moins un principe actif selon l'une quelconque des revendications 1 à 10 utiles pour la fabrication de médicaments traitant le diabète de type I, II et ses complications cardiovasculaires.

15. Compositions pharmaceutique selon la revendication 12 contenant au moins un principe actif selon l'une quelconque des revendications 1 à 10 utiles pour la fabrication de médicaments traitant le diabète de type I et II.

## Claims

1. Compounds of formula (I) : 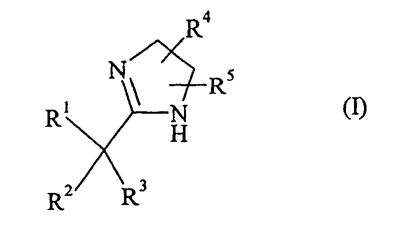 wherein :
• R¹ represents an optionally substituted heteroaryl group,
• R² represents an optionally substituted cycloalkyl group,
• R³ represents a hydrogen atom or an alkyl group, and
• R⁴ and R⁵, which may be identical or different, each represents a hydrogen atom, a halogen atom or an alkyl, polyhaloalkyl, R¹⁰―C(X)―R¹¹―, R¹⁰―Y―C(X)―R¹¹-, R¹⁰―C(X)―Y― R¹¹―, R¹⁰―Y―R¹¹― or R¹⁰―S(O)ₙ―R¹¹― group,
in which:
- R¹⁰ represents a hydrogen atom or an alkyl group,
- R¹¹ represents a bond, or an alkylene, alkenylene or alkynylene group,
- X represents an oxygen atom, a sulphur atom, or an NR¹² group in which R¹² represents a hydrogen atom or an alkyl group,
- Y represents an oxygen atom, a sulphur atom, or an amino or alkylamino group, and
- n represents an integer of from 1 to 2 inclusive,
the enantiomers, diastereoisomers and tautomers thereof, and also addition salts thereof with a pharmaceutically acceptable acid or base,
it being understood that:
- the term "alkyl" denotes a linear or branched hydrocarbon chain containing from 1 to 6 carbon atoms,
- the term "alkoxy" denotes an alkyl-oxy group in which the alkyl chain, which may be linear or branched, contains from 1 to 6 carbon atoms,
- the term "alkylene" denotes a linear or branched bivalent hydrocarbon chain containing from 1 to 6 carbon atoms,
- the term "alkenylene" denotes a linear or branched bivalent hydrocarbon chain containing from 1 to 6 carbon atoms and from 1 to 3 double bonds,
- the term "alkynylene" denotes a linear or branched bivalent hydrocarbon chain containing from 1 to 6 carbon atoms and from 1 to 3 triple bonds,
- the term "polyhaloalkyl" denotes a linear or branched carbon chain containing from 1 to 3 carbon atoms and from 1 to 7 halogen atoms,
- the term "heteroaryl" denotes a mono- or bi-cyclic group having from 5 to 11 ring members in which at least one of the rings is aromatic and containing in the monocycle or in the bicycle 1,2 or 3 hetero atoms selected from nitrogen, oxygen and sulphur, and
- the term "cycloalkyl" denotes a hydrocarbon monocycle or bicycle that contains from 3 to 10 carbon atoms and is optionally unsaturated by 1 or 2 unsaturated bonds,
- the expression "optionally substituted" associated with the terms cycloalkyl and heteroaryl denotes that the groups in question are unsubstituted or substituted by one or two identical or different substituents selected from halogen atoms and the groups alkyl, alkoxy, hydroxy, cyano, nitro, amino (optionally substituted by one or two alkyl groups) and -C(O)R_{d} wherein R_{d} represents a group selected from hydroxy, alkoxy and amino, it being understood that the heteroaryl group may be additionally substituted by an oxo group on the non-aromatic moiety of the heteroaryl.

2. Compound of formula (I) according to claim 1 wherein R⁴ and R⁵, which may be identical or different, each represents a hydrogen atom or an alkyl group, the enantiomers, diastereoisomers and tautomers thereof, and also addition salts thereof with a pharmaceutically acceptable acid or base.

3. Compound of formula (I) according to either claim 1 or claim 2 wherein R³ represents a hydrogen atom, the enantiomers, diastereoisomers and tautomers thereof, and also addition salts thereof with a pharmaceutically acceptable acid or base.

4. Compound of formula (I) according to any one of claims 1 to 3 wherein R¹ represents an optionally substituted heteroaryl group having 5 or 6 ring members, the enantiomers, diastereoisomers and tautomers thereof, and also addition salts thereof with a pharmaceutically acceptable acid or base.

5. Compound of formula (I) according to any one of claims 1 to 4 wherein R² represents a cyclopentyl, cyclohexyl or cycloheptyl group optionally substituted by an alkyl group, the enantiomers, diastereoisomers and tautomers thereof, and also addition salts thereof with a pharmaceutically acceptable acid or base.

6. Compound of formula (I) according to any one of claims 1 to 5 wherein R¹ represents an optionally substituted heteroaryl group having 5 or 6 ring members, R² represents a cyclohexyl or cycloheptyl group optionally substituted by an alkyl group, R³ represents a hydrogen atom and R⁴ and R⁵, which may be identical or different, each represents a hydrogen atom or an alkyl group, the enantiomers, diastereoisomers and tautomers thereof, and also addition salts thereof with a pharmaceutically acceptable acid or base.

7. Compound of formula (I) according to any one of claims 1, 2, 5 and 6 wherein the alkyl group is a methyl group, the enantiomers, diastereoisomers and tautomers thereof, and also addition salts thereof with a pharmaceutically acceptable acid or base.

8. Compound of formula (I) according to any one of claims 1 to 7 which is 2-[cyclohexyl(3-thienyl)methyl]-4-methyl-4,5-dihydro-1*H*-imidazole, the enantiomers, diastereoisomers and tautomers thereof, and also addition salts thereof with a pharmaceutically acceptable acid.

9. Compound of formula (I) according to any one of claims 1 to 7 which is (4*S*)-2-[cyclohexyl(3-thienyl)methyl]-4-methyl-4,5-dihydro-1*H*-imidazole, the enantiomers, diastereoisomers and tautomers thereof, and also addition salts thereof with a pharmaceutically acceptable acid.

10. Compound of formula (I) according to any one of claims 1 to 7 which is (4*R*)-2-[cyclohexyl(3-thienyl)methyl]-4-methyl-4,5-dihydro-1*H*-imidazole, the enantiomers, diastereoisomers and tautomers thereof, and also addition salts thereof with a pharmaceutically acceptable acid.

11. Process for the preparation of compounds of formula (I) according to claim 1, **characterised in that** there is used as starting material a compound of formula (II): 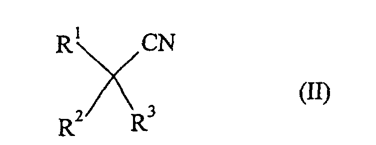 wherein R¹, R² and R³ are as defined for formula (I),
which compounds of formula (II) are condensed with a diamine (III) : 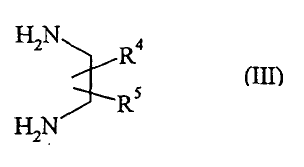 wherein R⁴ and R⁵ are as defined for formula (I)
to yield, in the presence of an appropriate catalyst, compounds of formula (I),
- which may optionally be purified according to a conventional purification technique,
- which, if desired, are separated into their stereoisomers according to a conventional separation technique,
- which, if desired, are converted into addition salts with a pharmaceutically acceptable acid or base,
it being understood that
- at any moment considered appropriate during the course of the process described above, the carbonyl, thiocarbonyl, amino or alkylamino group or groups of the starting reagents (II) and (III) may be protected and then, after condensation, deprotected for the purposes of the synthesis,
- the reagents (II) and (III) are prepared according to known procedures described in the literature.

12. Pharmaceutical composition comprising as active ingredient at least one compound according to any one of claims 1 to 10, alone or in combination with one or more pharmaceutically acceptable, inert, non-toxic excipients or carriers.

13. Pharmaceutical composition according to claim 12 comprising at least one active ingredient according to any one of claims 1 to 10 for use in the preparation of medicaments for the treatment of non-insulin-dependent type II diabetes, obesity, type I diabetes, hyperlipidaemia, hypercholesterolaemia and cardiovascular complications thereof.

14. Pharmaceutical composition according to claim 12 comprising at least one active ingredient according to any one of claims 1 to 10 for use in the preparation of medicaments for the treatment of type I and type II diabetes and cardiovascular complications thereof.

15. Pharmaceutical composition according to claim 12 comprising at least one active ingredient according to any one of claims 1 to 10 for use in the preparation of medicaments for the treatment of type I and type II diabetes.

## Patentansprüche

1. Verbindungen der Formel (I): 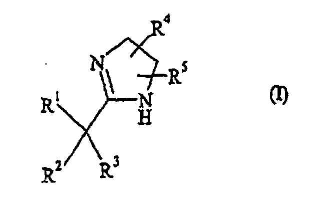 in der:
• R¹ eine gegebenenfalls substituierte Heteroarylgruppe bedeutet,
• R² eine gegebenenfalls substituierte Cycloalkylgruppe bedeutet,
• R³ ein Wasserstoffatom oder eine Alkylgruppe darstellt,
• R⁴ und R⁵, die gleichartig oder verschieden sind, ein Wasserstoffatom, ein Halogenatom oder eine Alkylgruppe, eine Polyhalogenalkylgruppe oder eine Gruppe R¹⁰)-C(X)-R¹¹-, R¹⁰-Y-C(X)-R¹¹-,R¹⁰-C(X)-Y-R¹¹-, R¹⁰-Y-R¹¹- oder R¹⁰-S(O)ₙ-R¹¹- bedeuten,
worin:
- R¹⁰ ein Wasserstoffatom oder eine Alkylgruppe bedeutet,
- R¹¹ eine Bindung oder eine Alkylen-, Alkenylen- oder Alkinylengruppe bedeutet,
- X ein Sauerstoffatom, ein Schwefelatom oder eine Gruppe NR¹² darstellt,
worin R¹² ein Wasserstoffatom oder eine Alkylgruppe bedeutet,
- Y ein Sauerstoffatom, ein Schwefelatom oder eine Amino- oder Alkylamino-Gruppe bedeutet, und
- n eine ganze Zahl mit einem Wert von 1 und 2 einschließlich bedeutet,
deren Enantiomere, Diastereoisomere, Tautomere sowie deren Additionssalze mit einer pharmazeutisch annehmbaren Säure oder Base, wobei es sich versteht, daß:
- der Begriff "Alkyl" für eine geradkettige oder verzweigte Kohlenwasserstoffgruppe steht, die 1 bis 6 Kohlenstoffatome enthält,
- der Begriff "Alkoxy" für eine Alkyl-oxygruppe steht, deren geradkettige oder verzweigte Alkylkette 1 bis 6 Kohlenstoffatome enthält,
- der Begriff "Alkylen" für eine geradkettige oder verzweigte, zweiwertige Kohlenwasserstoffkette steht, die 1 bis 6 Kohlenstoffatome enthält,
- der Begriff "Alkenylen" für eine geradkettige oder verzweigte, zweiwertige Kohlenwasserstoffkette steht, die 1 bis 6 Kohlenstoffatome und 1 bis 3 Doppelbindungen enthält,
- der Begriff "Alkinylen" für eine geradkettige oder verzweigte, zweiwertige Kohlenwasserstoffkette steht, die 1 bis 6 Kohlenstoffatome und 1 bis 3 Dreifachbindungen enthält,
- der Begriff "Polyhalogenalkyl" für eine geradkettige oder verzweigte Kohlenstoffkette steht, die 1 bis 3 Kohlenstoffatome und 1 bis 7 Halogenatome enthält,
- der Begriff "Heteroaryl" für eine monocyclische oder bicyclische Gruppe mit 5 bis 11 Kettengliedern steht, bei der mindestens einer der Ringe aromatisch ist und die in dem Monocyclus oder dem Bicyclus 1, 2 oder 3 Heteroatome ausgewählt aus Stickstoff, Sauerstoff und Schwefel, enthält, und
- der Begriff "Cycloalkyl" für einen Kohlenwasserstoff-Monocyclus oder -Bicyclus steht, der 3 bis 10 Kohlenstoffatome enthält und gegebenenfalls durch 1 oder 2 Unsättigungen ungesättigt ist,
- der Begriff "gegebenenfalls substituiert" in Bezug auf die Begriffe Cycloalkyl und Heteroaryl bedeutet, daß die betroffenen Gruppen nicht substituiert sind oder durch ein oder zwei gleichartige oder verschiedenartige Substituenten substituiert sind, die ausgewählt sind aus Halogenatomen und Alkyl-, Alkoxy-, Hydroxy-, Cyano-, Nitro-, (gegebenenfalls durch eine oder zwei Alkylgruppen substituierte) Amino-Gruppen und Gruppen -C(O)R_{d}, worin R_{d} eine Gruppe bedeutet ausgewählt aus Hydroxy, Alkoxy und Amino, mit der Maßgabe, daß die Heteroarylgruppe zusätzlich am nicht-aromatischen Teil des Heteroarylrestes durch eine Oxogruppe substituiert sein kann.

2. Verbindung der Formel (I) nach Anspruch 1, worin R⁴ und R⁵, die gleichartig oder verschieden sind, ein Wasserstoffatom oder eine Alkylgruppe bedeuten, deren Enantiomere, Diastereoisomere, Tautomere sowie deren Additionssalze mit einer pharmazeutisch annehmbaren Säure oder Base.

3. Verbindung der Formel (I) nach einem der Ansprüche 1 oder 2, worin R³ ein Wasserstoffatom bedeutet, deren Enantiomere, Diastereoisomere, Tautomere sowie deren Additionssalze mit einer pharmazeutisch annehmbaren Säure oder Base.

4. Verbindung der Formel (I) nach einem der Ansprüche 1 bis 3, worin R¹ eine Heteroarylgruppe mit 5 oder 6 Kettengliedern bedeutet, deren Enantiomere, Diastereoisomere, Tautomere sowie deren Additionssalze mit einer pharmazeutisch annehmbaren Säure oder Base.

5. Verbindung der Formel (I) nach einem der Ansprüche 1 bis 4, worin R² eine gegebenenfalls durch eine Alkylgruppe substituierte Cyclopentyl-, Cyclohexyloder Cycloheptyl-Gruppe bedeutet, deren Enantiomere, Diastereoisomere, Tautomere sowie deren Additionssalze mit einer pharmazeutisch annehmbaren Säure oder Base. deren Enantiomere,

6. Verbindung der Formel (I) nach einem der Ansprüche 1 bis 5, worin R¹ eine gegebenenfalls substituierte Heteroarylgruppe mit 5 oder 6 Kettengliedern bedeutet, R² eine gegebenenfalls durch eine Alkylgruppe substituierte Cyclohexyl- oder Cycloheptyl-Gruppe bedeutet, R³ ein Wasserstoffatom darstellt und R⁴ und R⁵, die gleichartig oder verschieden sind, ein Wasserstoffatom oder eine Alkylgruppe bedeuten, deren Enantiomere, Diastereoisomere, Tautomere sowie deren Additionssalze mit einer pharmazeutisch annehmbaren Säure oder Base.

7. Verbindung der Formel (I) nach einem der Ansprüche 1, 2, 5 oder 6, worin die Alkylgruppe eine Methylgruppe bedeutet, deren Enantiomere, Diastereoisomere, Tautomere sowie deren Additionssalze mit einer pharmazeutisch annehmbaren Säure oder Base.

8. Verbindung der Formel (I) nach einem der Ansprüche 1 bis 7, nämlich 2-[Cyclohexyl(3-thienyl)methyl]-4-methyl-4,5-dihydro-1*H*-imidazol, deren Enantiomere, Diastereoisomere, Tautomere sowie deren Additionssalze mit einer pharmazeutisch annehmbaren Säure oder Base.

9. Verbindung der Formel (I) nach einem der Ansprüche 1 bis 7, nämlich (4*S*)-2-[Cyclohexyl(3-thienyl)methyl]-4-methyl-4,5-dihydro-1*H*-imidazol, deren Enantiomere, Diastereoisomere, Tautomere sowie deren Additionssalze mit einer pharmazeutisch annehmbaren Säure oder Base.

10. Verbindung der Formel (I) nach einem der Ansprüche 1 bis 7, nämlich (4R)-2-[Cyclohexyl(3-thienyl)methyl]-4-methyl-4,5-dihydro-1*H*-imidazol, deren Enantiomere, Diastereoisomere, Tautomere sowie deren Additionssalze mit einer pharmazeutisch annehmbaren Säure oder Base.

11. Verfahren zur Herstellung der Verbindungen der Formel (I) nach Anspruch 1, **dadurch gekennzeichnet, daß** man als Ausgangsprodukt eine Verbindung der Formel (II) verwendet: 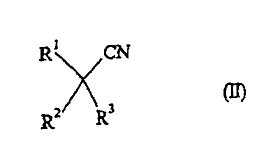 in der R¹, R²und R³ die bezüglich der Formel (I) angegebenen Bedeutungen besitzen, welche Verbindungen der Formel (II) man mit einem Diamin (III) kondensiert: 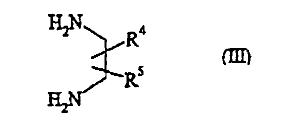 in der R⁴ und R⁵ die bezüglich der Formel (I) angegebenen Bedeutungen besitzen, so daß man in Gegenwart eines geeigneten Katalysators zu den Verbindungen der Formel (I) gelangt,
- welche gegebenenfalls mit Hilfe einer klassischen Reinigungsmethode gereingt werden können,
- welche man gegebenenfalls mit Hilfe einer klassischen Trennmethode in ihre Stereoisomeren auftrennt,
- welche man gewünschtenfalls in ihre Additionssalze mit einer pharmazeutisch annehmbaren Säure oder Base umwandelt, mit der Maßgabe,
- daß zu einem gegebenen Zeitpunkt im Verlaufe des oben beschriebenen Verfahrens die Carbonyl-, Thiocarbonyl-, Amino-, Alkylaminogruppe(n) der Ausgangsprodukte (II) und (III) geschützt und nach der Kondensation für die Zwecke der Synthese wieder von ihren Schutzgruppen befreit werden können,
- daß die Reaktionsteilnehmer (II) und (III) mit Hilfe von in der Literatur beschriebenen bekannten Verfahrensweisen hergestellt werden.

12. Pharmazeutische Zubereitung enthaltend als Wirkstoff mindestens eine Verbindung nach einem der Ansprüche 1 bis 10 allein oder in Kombination mit einem oder mehreren inerten, nicht-toxischen, pharmazeutisch annehmbaren Trägermaterialien oder Bindemitteln.

13. Pharmazeutische Zubereitung nach Anspruch 12 enthaltend mindestens einen Wirkstoff nach einem der Ansprüche 1 bis 10, für die Herstellung von Arzneimitteln zur Behandlung von nicht-insulinabhängigem Diabetes Typ II, der Fettsucht, Diabetes Typ I, Hyperlipidämie, Hypercholesterolämie und dadurch verursachten kardiovaskulären Komplikationen.

14. Pharmazeutische Zubereitung nach Anspruch 12 enthaltend mindestens einen Wirkstoff nach einem der Ansprüche 1 bis 10, für die Herstellung von Arzneimitteln zur Behandlung von Diabetes Typ I und II und dadurch verursachten kardiovaskulären Komplikationen.

15. Pharmazeutische Zubereitung nach Anspruch 12 enthaltend mindestens einen Wirkstoff nach einem der Ansprüche 1 bis 10, für die Herstellung von Arzneimitteln zur Behandlung von Diabetes Typ I und II.
